# EUROPEAN PATENT APPLICATION

(11) **EP 2 597 080 A1**
(43) Date of publication of application: **29.05.2013**
(21) Application number: 11809310.3
(22) Date of filing: 20.07.2011
(51) Int. Cl.: C07C 39/04, C07C 37/60, B01J 31/22

(54) **METHOD FOR THE CATALYTIC OXIDATION OF BENZENE TO PHENOL**

(30) Priority: 20.07.2010 ES 201031109
(71) Applicant: Universidad de Huelva, 21071 Huelva (ES)
(72) Inventor: PEREZ ROMERO, Pedro José, E-21071 Huelva (ES); DIAZ REQUEJO, María del mar, E-21071 Huelva (ES); CONDE ALCANTARA, Ana Isabel, E-21071 Huelva (ES)
(74) Representative: Temino Ceniceros, Ignacio
(86) International application number: PCT/ES2011/000236
(87) International publication number: WO 2012/010726

(57) **Abstract**

The invention relates to a method for the catalytic oxidation of benzene to phenol, where the oxidant is hydrogen peroxide and the reaction medium is a mixture of acetonitrile and water, the catalytic oxidation of the benzene being generated by a catalytic system based on copper compounds of formula Tp^{x}Cu(NCCH₃). Said method enables approximately 39% conversions of the starting hydrocarbons with phenol selectivities higher than 92% in processes taking place at 60°C with reaction times of between 2 and 4 hours.

## Description

### Field of the invention

The present invention falls in the field of chemistry, and more specifically in the field of the catalysis with application in reactions of oxidation of hydrocarbons.

### State of the prior art

Phenol is a product with an extraordinary importance in the chemical industry (Centi, G.; Perathoner, S. Catal. Today 2009, 143, 145). Its synthesis is carried out by means of the so-called cumene process, consisting of three consecutive reactions: (i) Friedel-Crafts alkylation of benzene with propene to give cumene, which (ii) is oxidized with oxygen to give cumyl hydroperoxide to, (iii) subsequently generate phenol and acetone in an acidic medium. The disadvantages of this process are several, highlighting a yield less than 10%, the obtaining of acetone as a by-product, and a relatively high energetic cost.

The first catalytic systems for this type of transformations are based on the Fenton reaction, employing Fe(II) and H₂O₂ in an acidic medium (Shilov, A. E.; Shul'pin, G. B. Chem. Rev. 1997, 97, 2879). In these conditions are generated hydroxyl radicals to which the oxidizing ability for the conversion of benzene to phenol has been attributed. However, the selectivity towards the latter is not very high because other collateral oxidation reactions occur that lead to the obtaining of a mixture of products (biphenyls, compounds with multiple hydroxyl groups and others of a polymeric nature).

A later variant of the Fenton system is the called Gif system, developed by the Barton group, which also is based on the use of iron and hydrogen peroxide catalysts in an acidic medium, but employing pyridine as an additive (Barton, D. H. R.; Doller, D. Acc. Chem. Res. 1992, 25, 504). This system is not effective for the oxidation of aromatic substrates, but for the aliphatic substrates.

On the basis of previous works, ENICHEM (R. Vignola, E. Battistel, D. Bianchi, R. Bortolo, R. Tassinari, EP 861688; Bianchi, D.; Bortolo, R.; Tassinari, R.; Ricci, M.; Vignola, R. Angew. Chem., Int. Ed. 2000, 39, 4321-4323) developed a catalytic system based on iron for the oxidation of benzene to phenol in a acetonitrile/water biphasic medium, in the presence of carboxylic acids. In the best conditions, this system provides conversions of 8-10% of benzene to phenol, with a 96% selectivity, which are, so far, the best values described for catalytic systems in homogeneous phase in the absence of acidic medium. Most of the described systems in homogeneous phase for this process employ an acid (glacial acetic or nitric) as a solvent (see Molinari, R.; Poeria, T. Asia-Pac. J. Chem. Eng. 2010, 5, 191-206 for this kind of systems as well as for others in heterogeneous phase).

Therefore, there is a need for an alternative chemical system that allows the direct conversion of benzene to phenol by an affordable oxidant.

### Explanation of the invention

The present invention describes a method for the direct conversion of benzene to phenol which overcomes the problems described in the State of the art.

Thus, in a first aspect the present invention provides a method for the catalytic oxidation of benzene to phenol where the oxidant is hydrogen peroxide and the reaction medium is a mixture of acetonitrile and water, and wherein the catalytic oxidation of the benzene is generated by a catalytic system comprising the general formula (1)

Tp^{x}M(NCCH₃) (1)

where Tp^{x} is a hydrotrispyrazolylborate ligand.

In a further aspect in particular, the catalytic system of general formula (1) comprises copper in the form of coordination compound, in oxidation state of +1.

In a further aspect in particular, the Tp^{x} ligand is selected from Tp^{Br3}, Tp^{Ph}, Tp^{Ms} Tp^{*,Br}, and Tp*

Said catalytic system promotes the oxidation of benzene to phenol, utilizing hydrogen peroxide as an oxidant, while the medium reaction is acetonitrile and water. Also, the benzoquinone, derived from an overoxidation of phenol, is obtained as a minority product.

In another aspect in particular, the method of the present invention is carried out at an operating temperature comprised between 40-80 °C; in a more particular aspect, the method of the present invention is carried out at a temperature of 60 °C.

In a further aspect in particular, the method of the present invention is carried out in a reaction time comprised between 1-4 hours; in a more particular aspect, the method of the present invention is carried out in a reaction time of 4 hours.

In a further aspect in particular, the synthesis of phenol is produced from benzene.

In a further aspect in particular, in the method of the present invention, the molar ratio between the catalyst and benzene is 1:200.

In a further aspect in particular, in the method of the present invention, the molar ratio between the benzene and hydrogen peroxide is 0.66.

In a further aspect in particular, in the method of the present invention, the reaction medium includes sulfolane as a co-solvent.

In a further aspect in particular, in the method of the present invention, the hydrogen peroxide is in an amount comprised between 0.8-25 mmol, in another more particular aspect, the amount of hydrogen peroxide in the method of the present invention is 1.5 mmol.

In a further aspect in particular, in the method of the present invention, the benzene is in an amount comprised between 1-15 mmol; in another more particular aspect, the amount of benzene is 1 mmol.

Thanks to the method thus described, there have reached conversions of the starting benzene close to 39% con phenol selectivities higher than 92%, in processes taking place at 60 °C with reaction times of 2-4 h.

### Detailed description of the invention. Embodiments and examples.

As stated above, the present invention provides a method for the direct conversion of benzene to phenol, employing hydrogen peroxide (30% v/v) as an oxidant, and a copper compound as catalyst, in a mixture of acetonitrile-water as a reaction medium.

The catalyst employed is a copper complex in oxidation state of +1 of general formula Tp^{x}Cu(NCCH₃), where Tp^{x} represents a ligand of the family of the hydrotrispyrazolylborates.

In particular, the complexes with Tp^{Br3}, Tp^{Ph}, Tp^{Ms} Tp^{*,Br}, and Tp* ligands as shown below, in the formulation [2], are those which have provided the best results. The synthesis of these compounds has already been described (see Mairena, M. A.; Urbano, J.; Carbajo, J.; Maraver, J. J.; Alvarez, E.; Diaz-Requejo, M. M.; Pérez, P. J. Inorg. Chem. 2007, 46, 7428-7435 as well as the references included in this work).

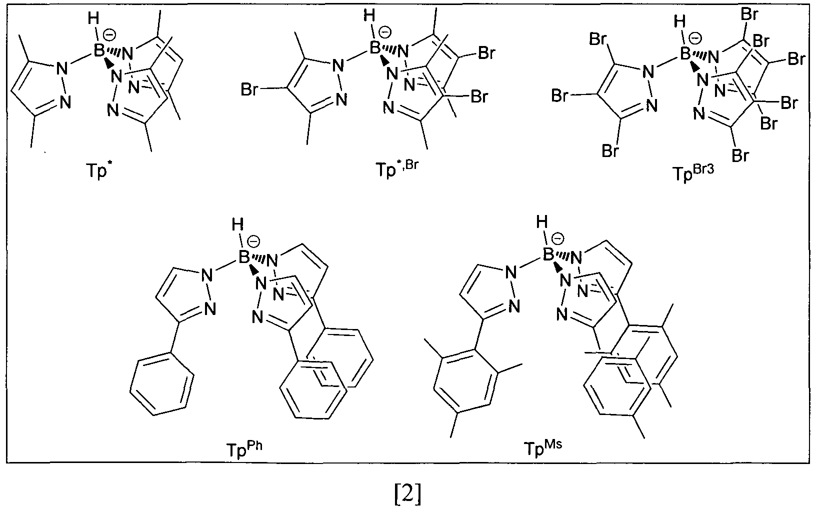

This simple system consisting of the complex of copper, benzene, hydrogen peroxide (30% v/v), water, and acetonitrile has led to a 33% conversion of benzene with phenol selectivity of 85%, without requiring the use of any additives (acids, phase-transfer agents, or the like).

The used catalyst can be any of the complexes Tp^{x}Cu(NCCH₃), Tp^{x} being a ligand of those shown in the formulation [2].

The two-phase system arranges the reagents and products in the following way: the acetonitrile includes benzene, catalyst and, mostly, phenol which is formed while the aqueous phase only contains the hydrogen peroxide and a small fraction of phenol which is formed.

It has been proven that the acetonitrile is the most effective organic solvent; for the purposes of yield and selectivity it is considered a determinant component for the particular reaction system.

The reaction takes place in acetonitrile, being 2 mL the preferred total volume. The water volume corresponds to which accompanies the hydrogen peroxide used in each experiment, and which corresponds to a commercial solution of 30% v/v.

The amount of benzene used may vary between 1 and 15 mmol, being preferred 1 mmol.

The amount of hydrogen peroxide used may vary between 0.8 and 25 mmol, being preferred 1.5 mmol.

The reagents, benzene and hydrogen peroxide may be present in the reaction mixture according to a molar ratio comprised between 0.2 and 1.25, being preferred 0.66.

The catalyst loading may vary between 0.1% and 0.5% with respect to benzene, being preferred 0.5%.

### EXAMPLE 1

Into a 25 mL flask, 0.005 mmol of the complex Tp^{*,Br}Cu(NCCH₃) were dissolved in 2 mL of acetonitrile, and 1 mmol of benzene and 1.5 mmol of hydrogen peroxide were added. Into the flask was placed a reflux column, and the mixture was stirred for 8 h heating at 60 °C. At the end of the reaction, the reaction mixture was extracted with chloroform (1.5-2 mL). The concentration of the products in this organic phase was determined by RMN, taking an aliquot of the same, and using diethyl malonate as internal standard. The Table 1 contains the results from three experiments, carried out by varying the amount added of oxidant. The conversion of benzene represents the fraction consumed of benzene. The phenol selectivity corresponds to the percentage (mol%) of benzene converted to phenol, the rest up to 100% has been identified as benzoquinone.

**Table 1**

| Entry | mmol C₆H₆ | mmol H₂O₂ | Conversion C₆H₆ (mol%) | Phenol selectivity (mol%) |
|---|---|---|---|---|
| 1 | 1 | 1.5 | 18 | 78 |
| 2 | 1 | 2.5 | 17 | 82 |
| 3 | 1 | 5 | 26 | 73 |

### EXAMPLE 2

To evaluate the effect of the benzene:hydrogen peroxide ratio, the above method was repeated using Tp^{*,Br}Cu(NCCH₃) as a catalyst, and two reaction times, 2 h and 4 h, heating at 60 °C. The results are shown in Tables 2 and 3, respectively.

**Table 2**

| Entry | mmol of H₂O₂ | Reaction time (h) | Conversion C₆H₆ (mol%) | Phenol selectivity (mol%) |
|---|---|---|---|---|
| 1 | 1.5 | 2 | 19 | 80 |
| 2 | 2.5 | 2 | 20 | 80 |
| 3 | 5 | 2 | 15 | 73 |

**Table 3**

| Entry | mmol of H₂O₂ | Reaction time (h) | Conversion C₆H₆ (mol%) | Phenol selectivity (mol%) |
|---|---|---|---|---|
| 1 | 0.8 | 4 | 11 | 91 |
| 2 | 1 | 4 | 30 | 87 |
| 3 | 1.5 | 4 | 33 | 85 |
| 4 | 2.5 | 4 | 30 | 80 |
| 5 | 5 | 4 | 19 | 79 |

### EXAMPLE 3

The method described in example 1 was repeated using different complexes Tp^{x}Cu(NCCH₃) as catalysts, heating the reaction mixture for 4 h at 60 °C. The results are shown in Tables 4 and 5, for two different ratios of benzene and hydrogen peroxide.

**Table 4**

| Entry | Catalyst | mmol C₆H₆ | mmol H₂O₂ | Conversion C₆H₆ (mol%) | Phenol selectivity (mol%) |
|---|---|---|---|---|---|
| 1 | Tp^{*,Br}Cu(NCCH₃) | 1 | 5 | 19 | 79 |
| 2 | Tp^{*}Cu(NCCH₃) | 1 | 5 | 15 | 73 |

**Table 5**

| Entry | Catalyst | mmol C₆H₆ | mmol H₂O₂ | Conversion C₆H₆ (mol%) | Phenol selectivity (mol%) |
|---|---|---|---|---|---|
| 1 | Tp^{*}Cu(NCCH₃) | 1 | 1.5 | 19 | 79 |
| 2 | Tp^{*,Br}Cu(NCCH₃) | 1 | 1.5 | 33 | 85 |
| 3 | Tp^{Ms}Cu(NCCH₃) | 1 | 1.5 | 27 | 78 |
| 4 | Tp^{Ph}Cu(NCCH₃) | 1 | 1.5 | 27 | 85 |
| 5 | Tp^{Br3}Cu(NCCH₃) | 1 | 1.5 | 18 | 72 |

### EXAMPLE 4

To evaluate the effect of the temperature, the above method was repeated using Tp^{*,Br}Cu(NCCH₃) as a catalyst, and 1.5 mmol of H₂O₂ as an oxidant, at different temperatures. The results are shown in Table 6.

**Table 6**

| Entry | Temperature (°C) | Time (h) | Conversion C₆H₆ (mol%) | Phenol selectivity (mol%) |
|---|---|---|---|---|
| 1 | 20 | 4 | <1 | -- |
| 2 | 40 | 4 | 26 | 77 |
| 3 | 60 | 4 | 33 | 85 |
| 4 | 80 | 4 | 11 | 91 |
| 5 | 80 | 2 | 25 | 92 |

### EXAMPLE 5

0.015 mmol of the complex Tp^{*,Br}Cu(NCCH₃) were dissolved in 6 mL of acetonitrile, and 3 mmol of benzene and 4.5 mmol of hydrogen peroxide (0.45 mL of 30% v/v in water) were added. After stirring for 4 h at 60 °C, the analysis of the reaction mixture led to the results that appear in the Table 7.

The experiment of the entry 2 corresponds to that of the entry 1 multiplied by three in all its components, in order to scale the reaction.

**Table 7**

| Entry | mmol C₆H₆ | mmol H₂O₂ | Catalyst (mmol) | mL of MeCN | Conversion C₆H₆ (mol%) | Phenol selectivity (mol%) |
|---|---|---|---|---|---|---|
| 1 | 3 | 4.5 | 0.015 | 6 | 21 | 82 |
| 2 | 9 | 13.5 | 0.045 | 12 | 11 | 81 |

### EXAMPLE 6

The above method was repeated with Tp^{x}Cu(NCCH₃), by varying the concentration of the catalyst on the reaction, using different amounts of benzene, hydrogen peroxide and acetonitrile in the mixture. After stirring for 1 h at 80 °C, the analysis of the reaction mixture after this time led to the results that appear in the Tables 8 and 9, where the complexes Tp*Cu(NCMe) and Tp*^{,Br}Cu(NCMe) have been employed as catalysts, respectively.

**Table 8. Catalyst Tp*Cu(NCMe)**

| Entry | mmol C₆H₆ | mmol H₂O₂ | Catalyst (mmol) | mL of MeCN | Conversion C₆H₆ (mol%) | Phenol selectivity (mol%) |
|---|---|---|---|---|---|---|
| 1 | 3 | 4.5 | 0.015 | 3 | 17 | 80 |
| 2 | 10 | 15 | 0.015 | 4 | 10 | 85 |
| 3 | 10 | 20 | 0.015 | 4 | 14 | 85 |

**Table 9. Catalyst Tp*^{,Br}Cu(NCMe)**

| Entry | mmol C₆H₆ | mmol H₂O₂ | Catalyst (mmol) | mL of MeCN | Conversion C₆H₆ (mol%) | Phenol selectivity (mol%) |
|---|---|---|---|---|---|---|
| 1 | 10 | 15 | 0.015 | 4 | 12 | 80 |
| 2 | 10 | 20 | 0.015 | 4 | 12 | 81 |
| 3 | 10 | 25 | 0.01 | 3 | 12 | 88 |
| 4 | 15 | 10 | 0.015 | 4 | 7 | 80 |

### EXAMPLE 7

According to the works of Ricci *et al.* (Bianchi, D.; Balducci, L.; Bortolo, R.; D'Aloisio, R.; Ricci, M.; Span , G.; Tassinari, R.; Tonini, C.; Ungarellia, R. Adv. Synth. Catal. 2007, 349, 979-986, and references included in this work), the addition of sulfolane (C₄H₈SO₂) to the reaction medium allows to increase the selectivity of the products to the phenol. To evaluate the effect of this additive, acetonitrile-sulfolane mixtures have been used as reaction medium.

Into a 25 ml flask, 0.005 mmol of the complex Tp^{*,Br}Cu(NCCH₃) were dissolved in 2 mL of acetonitrile, and 1 mmol of benzene, 4 mmol of sulfolane, and finally, 1.5 mmol of hydrogen peroxide (0.15 mL of 30% v/v in water) were added. Into the flask was placed a reflux column, and the mixture was stirred for 4 h at 60 °C. At the end of the reaction, the reaction mixture was allowed to cool at room temperature and extracted with chloroform (1.5-2 ml); the concentration of the oxidation products in the organic phase was determined by RMN, taking an aliquot of the same, and using diethyl malonate as internal standard.

**Table 10**

| Entry | Catalyst | Time (h) | C₆H₆/sulfolane (1:4 mmol) | MeCN/sulfolane (2/0.38 mL) | Conversion C₆H₆ (mol%) | Phenol selectivity (mol%) |
|---|---|---|---|---|---|---|
| 1 | Tp^{*,Br}Cu(NCCH₃) | 4 | 1:4 | 2/0.38 | 14 | 93 |

### EXAMPLE 8

The above method was repeated employing different complexes Tp^{x}Cu(NCCH₃) as a catalyst, heating the reaction mixture for 2 h at 80 °C. The results are shown in Table 11.

**Table 11**

| Entry | Catalyst | Time (h) | C₆H₆/sulfolane (1:4 mmol) | MeCN/sulfolane (2/0.38 mL) | Conversion C₆H₆ (mol%) | Phenol selectivity (mol%) |
|---|---|---|---|---|---|---|
| 1 | Tp^{*,Br}Cu(NCCH₃) | 2 | 1:4 | 2/0.38 | 16 | 94 |
| 2 | Tp^{Ms}Cu(NCCH₃) | 2 | 1:4 | 2/0.38 | 23 | 83 |
| 3 | Tp^{Ph}Cu(NCCH₃) | 2 | 1:4 | 2/0.38 | 24 | 92 |
| 4 | Tp^{Br3}Cu(NCCH₃) | 2 | 1:4 | 2/0.38 | 34 | 91 |

### EXAMPLE 9

The above method was repeated employing different complexes Tp^{x}Cu(NCCH₃) as a catalyst, heating the reaction mixture for 2 h at 80 °C, by varying the amounts of H₂O₂. The results are shown in Table 12.

**Table 12**

| Entry | Catalyst | Time (h) | mmol H₂O₂ | C₆H₆/sulfolane (1:4 mmol) | MeCN/sulfolane (2/0.38 mL) | Conversion C₆H₆ (mol%) | Phenol selectivity (mol%) |
|---|---|---|---|---|---|---|---|
| 1 | Tp^{Ph}Cu(NCCH₃) | 2 | 1.5 | 1:4 | 2/0.38 | 24 | 92 |
| 2 | | 2 | 2.5 | 1:4 | 2/0.38 | 33 | 85 |
| 3 | | 2 | 5 | 1:4 | 2/0.38 | 28 | 89 |
| 4 | Tp^{Br3}Cu(NCCH₃) | 2 | 1.5 | 1:4 | 2/0.38 | 34 | 91 |
| 5 | | 2 | 2.5 | 1:4 | 2/0.38 | 39 | 92 |

By way of comparison, the experiment of the entry 5 has been repeated, but without the addition of sulfolane:

**Table 13**

| Entry | T(°C) | Time (h) | mmol H₂O₂ | Conversion C₆H₆ (mol%) | Phenol selectivity (mol%) |
|---|---|---|---|---|---|
| 1 | 80 | 2 | 2.5 | 17 | 71 |

### EXAMPLE 10

To evaluate the effect of the benzene:hydrogen peroxide ratio, the above method was repeated using Tp^{Br3}Cu(NCCH₃) as a catalyst, heating the reaction mixture for 2 h at 80 °C. The results are shown in Table 14.

**Table 14**

| Entry | mmol C₆H₆ | mmol H₂O₂ | Catalyst (mmol) | mL of MeCN | Sulfolane (mmol) | Conversion C₆H₆ (mol%) | Phenol selectivity (mol%) |
|---|---|---|---|---|---|---|---|
| 1 | 10 | 15 | 0.015 | 4 | 5 | 12 | 83 |
| 2 | 10 | 25 | 0.01 | 3 | 10 | 15 | 87 |
| 3 | 10 | 25 | 0.01 | 4 | 20 | 14 | 91 |

## Claims

1. Method for the catalytic oxidation of benzene to phenol, where the oxidant is hydrogen peroxide and the reaction medium is a mixture of acetonitrile and water, **characterized in that** the catalytic oxidation of the benzene is generated by a catalytic system comprising the general formula (1)
Tp^{x}Cu(NCCH₃) (1)
where Tp^{x} is a hydrotrispyrazolylborate ligand.

2. Method according to claim 1, where the Tp^{x} ligand of the catalytic system is selected from Tp^{Br3}, Tp^{Ph}, Tp^{Ms} Tp^{*,Br}, and Tp*.

3. Method according to any of the claims 1-2, where the operating temperature is comprised between 40-80°C.

4. Method according to any of the preceding claims, where reaction time is comprised between 1-4 hours.

5. Method according to any of the preceding claims, where the synthesis of phenol is produced from benzene.

6. Method according to the preceding claims, where the molar ratio between the catalyst and benzene is 1:200.

7. Method according to any of the preceding claims, where the molar ratio between the benzene and hydrogen peroxide is 0.66.

8. Method according to claims 1 to 8, wherein the reaction medium includes sulfolane as a co-solvent.

9. Method according to any of the preceding claims, where the hydrogen peroxide is in an amount comprised between 0.8-25 mmol.

10. Method according to any of the preceding claims, where the benzene is in an amount comprised between 1-15 mmol.
